# EUROPÄISCHE PATENTANMELDUNG

(11) **EP 1 008 851 A1**
(43) Veröffentlichungstag der Anmeldung: **14.06.2000**
(21) Anmeldenummer: 99124751.1
(22) Anmeldetag: 13.12.1999
(51) Int. Cl.: G01N 33/50, G01N 33/92

(54) **Hautpflege-System**

(30) Priorität: 11.12.1998 DE 19857349
(71) Anmelder: Schnizer, Wolfgang, Prof. Dr. med., 80687 München (DE)
(72) Erfinder: Schnizer, Wolfgang, Prof. Dr. med., 80687 München (DE)
(74) Vertreter: Koepe, Gerd L., Dipl.-Chem.

(57) **Zusammenfassung**

Die Erfindung betrifft ein System zur Ermittlung des Gehalts der Haut, insbesondere der menschlichen Haut, an verschiedenen, insbesondere an für die Funktionen der Haut essentiellen, Inhaltsstoffen und zur an das Ergebnis der Ermittlung angepaßten Supplementierung mit den fehlenden Inhaltsstoffen, gekennzeichnet durch Mittel zur Ermittlung des Gehalts der Haut eines Individuums an vorgegebenen Inhaltsstoffen; Mittel zum Vergleich der ermittelten Werte mit Standardwerten des Gehalts der Haut an vorgegebenen Inhaltsstoffen; Mittel zur Ableitung des Bedarfs der Haut des Individuums an den vorgegebenen Inhaltsstoffen aufgrund des Vergleichs der ermittelten Werte mit den Standardwerten; Mittel zur Komposition einer Zubereitung, deren Zusammensetzung exakt auf den Bedarf der Haut an den vorgegebenen Inhaltsstoffen abgestimmt ist; und Mittel zur Supplementierung der Haut des Individuums mit der komponierten Zubereitung.

## Beschreibung

Die vorliegende Erfindung betrifft ein System zur Ermittlung des Gehalts der menschlichen Haut an verschiedenen, für die natürlichen Funktionen der Haut essentiellen Inhaltsstoffen und zur an das Ergebnis der Ermittlung angepaßten Supplementierung mit den fehlenden Inhaltsstoffen. Die Erfindung betrifft auch ein Verfahren zur Behandlung und/oder Pflege der Haut und zur Vorbeugung von Mangelerscheinungen daran, insbesondere ein kosmetisches Behandlungsverfahren, kosmetisches Pflegeverfahren und kosmetisches Vorbeugeverfahren.

Das Erfordernis, daß die menschliche Haut gesund ist und ihre natürlichen Funktionen optimal erfüllen kann, setzt voraus, daß sie in allen Bereichen ihren Funktionen entsprechend aufgebaut ist und nicht durch Mängel an im natürlichen Fall vorhandenen Inhaltsstoffen Fehlfunktionen auftreten oder sogar bestimmte Funktionen mangels der natürlicherweise vorhandenen Hautkomponenten gar nicht erfüllt werden können.

Beispielsweise ist eine besonders wichtige Funktion der Haut die Barriere-Funktion gegen Wasserverlust. Wasserhaltung und -permeabilität sind in diesem Zusammenhang zwei wichtige Kenngrößen. Ist das Wechselspiel dieser Kenngrößen gestört, kommt es relativ schnell zu Veränderungen des Hautstatus, die Krankheits-Werte erreichen können. Dies hat auch erhebliche Auswirkungen auf die notwendige Flexibilität der Haut, deren mechanische Stabilität sowie den Abschuppungsprozeß der Hornschicht (stratum corneum).

Für die Barrierefunktion der Haut ist in erster Linie die Hornschicht und deren Struktur verantwortlich. Diese wird stark von den in diese Schicht eingelagerten Fettstoffen (Lipiden) beeinflußt. Die auf der Hautoberfläche vorkommenden Talgdrüsen-Lipide spielen im Gegensatz zu den Stramm corneum-Lipiden für die Barrierefunktion der Haut nur eine untergeordnete Rolle.

Entscheidend ist ein Gemisch aus Cholesterol, Ceramiden und freien Fettsäuren, die intercellulär der Ausbildung von lamellären Strukturen ("laminar bilayers") dienen (N. S. Schuser, P. M. Elias, Adv. Lipid Res. 24 (1991), 27 - 56; P. M. Elias, K. R. Feingold, Semin. Dermatol. 11(1992), 176 - 182; P. M. Elias, G. K. Menon, Adv. Lipid Res. 24 (1991), 1 - 26). In derartigen Laminar-Strukturen findet sich die Basis der Barrierefunktion der Haut, die aktiv durch Stoffwechsel-Leistungen aus tieferen Hautschichten unterhalten wird.

Kommt es zu einem Mangel oder einer Störung der physiologischen Lipid-Muster, wird die Barrierefunktion beeinträchtigt. Dies äußert sich z. B. in einem erhöhten transepidermalen Wasserverlust. Das auffälligste Symptom ist dann die Haut-Trockenheit. Eine fettarme Haut neigt zu Trockenheit, wird rissig, spröde, schuppig und disponiert zu Juckreiz und Infektionen. Diese Symptomatik ist Teil einer Reihe von Hauterkrankungen und wird u. a. mit dem Fehlen normaler Verhältnisse der Stratum corneum-Lipide in Verbindung gebracht (C. Melnik, Biochemie und Pathobiologie des epidermalen Lipidstoffwechsels, G. Thieme-Verlag Stuttgart, New York 1990). Dazu gehören z. B. chronische Ekzeme, hier besonders Neurodermitis, Ichthyosis, Psoriasis, konstitutionelle Bereitschaft zur trockenen Haut, trockene Altershaut, berufsbedingte Kontakte mit die Fette herauslösenden organischen Lösungsmitteln usw..

Die Lipide der Hornschicht sind nach Qualität und Quantität durch chemische Analysen darstellbar. Das notwendige Material für die chemische Analyse kann z. B. entweder durch Herauslösen aus der Haut durch geeignete Lösungsmittel oder durch Abrisse der Hornschicht der Haut (sog. "Stripping") gewonnen werden. Dabei hat sich gezeigt, daß die Lipidmuster der Haut von Individuum zu Individuum verschieden sind und auch bei einem Individuum Schwankungen unterliegen, die nicht nur vom aktuellen gesundheitlichen Status, vom Alter und/oder der aktuellen Behandlung der Haut abhängen, sondern auch in unterschiedlichen Regionen der menschlichen Haut verschieden sind (M. A. Lampe et al., J. Lipid Res. 24 (1983), 120 - 130; P. M. Elias et al., J. Invest. Dermatol. 76 (1981), 297 - 301; N. Yoshikawa et al., Dermatology 188 (1994), 207 - 214).

Unzureichende Lipid-Verhältnisse disponieren oder führen zu Schädigungen bzw. Krankheitszuständen der Haut. Erwünscht ist, daß es durch rechtzeitige Versorgung der Haut mit den fehlenden Inhaltsstoffen gar nicht erst zu solchen Schädigungen bzw. Krankheitszuständen der Haut kommt. Sollten sie jedoch tatsächlich auftreten, kann die topische Applikation eines den physiologischen Bedingungen entsprechenden und diese wiederherstellenden Lipidgemischs die Barrierefunktion wieder verbessern. Insbesondere bestehen positive Erkenntnisse dahingehend, daß nach experimentell akut (beispielsweise chemisch oder mechanisch) beeinträchtigter Barrierefunktion, die auf eine Störung der Lipid-Verhältnisse zurückzuführen ist, die externe Lipid-Applikation eine viel schnellere Beseitigung der Schädigung bewirkt als eine natürliche Regeneration ohne externe Lipid-Applikation (M. Mao-Quiang et al., Arch. Dermatol. 131 (1995), 809 - 816; L. Yang et al., Br. J. Dermatol. 133 (1995), 679 - 685; M. Mao-Quiang et al., Arch. Dermatol. 129 (1993), 728 - 738; E. Zettersten et al., J. Am. Acad. Dermatol. 37 (1997), 403 - 408; M. Mao.Quiang et al., J. Invest. Dermatol. 106 (1996), 1096 - 1111; M. S. Imokawa et al., J. Invest. Dermatol. 87 (1986), 758 - 761): Physiologisch zusammengesetzte Lipid-Gemische beschleunigen die für eine Restitution zuständigen Stoffwechsel-Prozesse. Ähnliche Befunde liegen für die Altershaut vor (E. Zettersten et al., J. Am. Acad. Dermatol. 37 (1997), 403 - 408). Der Terminus "physiologisch zusammengesetzt" bedeutet in diesem Zusammenhang eine Orientierung an Verhältnissen in gesunder ("normaler") Haut.

Interessant ist in diesem Zusammenhang die Beobachtung, daß die externe Applikation von einzelnen Lipidkomponenten (selbst wenn sich diese bei der Untersuchung als fehlend erweisen) oder auch von Lipid-Gemischen mit einer von der speziellen Hautlipid-Zusammensetzung abweichenden Lipid-Zusammensetzung oftmals die Restitution der Haut eher hemmen oder zumindest störend beeinträchtigen und es nur schleppend zur Erholung der Haut unter Wiederherstellung der Barrierefunktion kommt (Lit.: s. o.). Andererseits kennt man Hauterkrankungen, bei denen aufgrund von Hautstoffwechsel-Anomalien einzelne Lipide oder begrenzte Klassen von Lipiden in der Haut fehlen oder unterrepräsentiert sind. Bei diesen Erkrankungen kann die externe Substitution allein der fehlenden Lipide eine Besserung des Zustandes der Haut hervorrufen oder zumindest fördern. Ein Beispiel hierfür ist die Neurodermitis, bei der offenbar u. a. ein Mangel an γ-Linolensäure vorliegt (J. M. Janossi et al., Z. Hautkr. 70 (1995), 498 - 502).

Möglicherweise war diese uneinheitliche Verhaltensweise der Haut bei verschiedenen Lipid-Mangelsituationen der Grund dafür, daß bisher extern anwendbare Zubereitungen physiologischer Lipid-Mixturen mit einer qualitativ und quantitativ geeigneten Lipidzusammensetzung nicht realisiert wurden. Genausowenig wurde die Zusammensetzung von Lipidzubereitungen für die externe Anwendung auf die Ergebnisse der Ermittlung der tatsächlichen Zusammensetzung des Lipid-Spektrums und die wünschenswerterweise zu supplementierenden Lipide abgestimmt.

Was vorstehend am Beispiel der Lipide der Haut dargestellt wurde, gilt natürlich in gleicher Weise auch für andere Inhaltsstoffe, wie beispielsweise Spurenelemente, Proteine, Vitamine, Enzyme, Aminosäuren und/oder andere, in gesunder Haut vorkommende Komponenten.

Der Erfindung liegt daher die Aufgabe zugrunde, ein System zur Ermittlung des Gehalts der menschlichen Haut an verschiedenen für die natürlichen Funktionen der Haut essentiellen Inhaltsstoffen wie beispielsweise Lipiden, Spurenelementen, Proteinen, Vitaminen, Enzymen, Aminosäuren und/oder anderen, in gesunder Haut vorkommenden Komponenten und zur an das Ergebnis der Ermittlung angepaßten Supplementierung mit dem/den fehlenden Inhaltsstoff(en) bereitzustellen. Insbesondere ist es Aufgabe der Erfindung, ein System bereitzustellen, das es ermöglicht, schnell und zuverlässig den Gehalt der Haut an für die Funktionen der Haut essentiellen Inhaltsstoffen zu analysieren und mit dem Analyseergebnis eine Grundlage für die Zusammenstellung einer individuellen Zubereitung bereitstellen zu können, mit der der Haut auf topischem Weg die fehlenden essentiellen Komponenten zugeführt werden können.

Weiter ist es Aufgabe der Erfindung, ein System zur Verfügung zu stellen, mit dem es einfach und schnell möglich ist, den Gehalt der Haut an Inhaltsstoffen zu analysieren, die für zu erwartende zukünftige Belastungen wie beispielsweise (Sonnen-) Strahlungsbelastung oder chemische Belastung (Kontakt mit Lösungsmitteln; Salzwasser o. ä.) wünschenswert sein können, um die Belastung besser ertragen oder mildern zu können, und mit dem der Haut anschließend die gewünschten, aber nicht vorhandenen Inhaltsstoffe zugeführt werden können und damit ein gesunder Hautstatus sichergestellt oder wiederhergestellt werden kann. Eine weitere Aufgabe der Erfindung war, ein Verfahren zur Behandlung und/oder Pflege der Haut oder zur Vorbeugung von Mangelerscheinungen daran bereitzustellen. Im Rahmen dieses Verfahrens sollten der Gehalt der Haut an Inhaltsstoffen schnell und zuverlässig analysiert werden können und Defizite an solchen Inhaltsstoffen schnell und gezielt behoben werden können, bevor Mangelerscheinungen auftreten, um einen gesunden, alle Inhaltsstoffe präsentierenden Status der Haut erreichen zu können. Insbesondere war es Aufgabe der Erfindung, ein kosmetisches Behandlungsverfahren, kosmetisches Pflegeverfahren und kosmetisches Vorbeugeverfahren mit den vorgenannten Zielen zu schaffen.

Überraschenderweise wurde nämlich gefunden, daß mit dem System gemäß der Erfindung zuverlässig der Gehalt der menschlichen Haut an essentiellen Inhaltsstoffen bestimmt werden kann und das Ergebnis dazu verwendet werden kann, die Komponenten topisch anwendbarer Zubereitungen exakt darauf einzustellen, welche Komponenten der Haut zugeführt werden müssen. Damit war ist zum ersten Mal möglich, auf spezielle Erfordernisse zu reagieren, sei es bei der Zufuhr eines ganzen Spektrums zuzuführender Komponenten (wie beispielsweise bei der Supplementierung der Haut mit den Lipiden, die die Barrierefunktion der besonderen Haut wieder restituieren), sei es bei der Zufuhr einzelner, besonderer Komponenten (wie beispielsweise γ-Linolensäure bei Neurodermitis oder Vitamin E bei dem Ziel, die Haut besser vor zukünftiger Sonnenbestrahlung zu schützen).

Die Erfindung betrifft ein System zur Ermittlung des Gehalts der Haut an verschiedenen, insbesondere an für die Funktionen der Haut essentiellen, Inhaltsstoffen und zur an das Ergebnis der Ermittlung angepaßten Supplementierung mit den fehlenden Inhaltsstoffen, gekennzeichnet durch
- Mittel zur Ermittlung des Gehalts der Haut eines Individuums an vorgegebenen Inhaltsstoffen;
- Mittel zum Vergleich der ermittelten Werte mit Standardwerten des Gehalts der Haut an vorgegebenen Inhaltsstoffen;
- Mittel zur Ableitung des Bedarfs der Haut des Individuums an den vorgegebenen Inhaltsstoffen aufgrund des Vergleichs der ermittelten Werte mit den Standardwerten;
- Mittel zur Komposition einer Zubereitung, deren Zusammensetzung exakt auf den Bedarf der Haut an den vorgegebenen Inhaltsstoffen abgestimmt ist; und
- Mittel zur Supplementierung der Haut des Individuums mit der komponierten Zubereitung.

Insbesondere findet das erfindungsgemäße System Anwendung bei menschlicher Haut. Denkbar ist es jedoch auch, daß eine Anwendung bei tierischer Haut, insbesondere Säuger-Haut, erfolgt.

Aufgrund des Pflege-Charakters oder Präventiv-Charakters schließt das erfindungsgemäße System auch den Fall ein, daß sich mit dem Mittel zur Ermittlung des Gehalts der Haut eines Individuums an vorgegebenen Inhaltsstoffen ergibt, daß der Hautstatus mehr oder weniger den idealen Verhältnissen einer gesunden Haut entspricht. In diesem Fall ist die Abweichung der ermittelten Werte von den Standardwerten des Gehalts der Haut an vorgegebenen Inhaltsstoffen gering oder sogar gleich Null. In diesem Fall kann das System gemäß der Erfindung zu Pflege- oder Präventionszwecken Mittel zur Supplementierung der Haut des Individuums mit Zubereitungen umfassen, die ein zukünftiges Abweichen der Werte des Gehalts der Haut des Individuums an den in Rede stehenden Inhaltsstoffen verhindern oder zumindest dazu beitragen, ein zukünftiges Abweichen zu verhindern.

Die Erfindung betrifft auch ein Verfahren, insbesondere ein kosmetisches Verfahren, zur Behandlung und/oder Pflege der Haut und/oder zur Vorbeugung von Mangelerscheinungen daran, bei dem man den Gehalt der Haut eines Individuums an vorgegebenen Inhaltsstoffen ermittelt; die ermittelten Werte mit Standardwerten des Gehalts der Haut an vorgegebenen Inhaltsstoffen vergleicht; aufgrund des Vergleichs der ermittelten Werte mit den Standardwerten den Bedarf des Individuums an den vorgegebenen Inhaltsstoffen ableitet; eine Zubereitung zusammenstellt, deren Zusammensetzung exakt auf den Bedarf der Haut an den vorgegebenen Inhaltsstoffen abgestimmt ist; und die Haut des Individuums mit der zusammengestellten Zubereitung supplementiert.

Insbesondere betrifft die Erfindung ein Verfahren, das an und/oder auf der menschlichen Haut angewandt wird.

Aufgrund des Pflege-Charakters oder Präventiv-Charakters schließt das erfindungsgemäße Verfahren, insbesondere das kosmetische Verfahren, auch den Fall ein, daß sich in dem ersten Verfahrensschritt bei der Ermittlung des Gehalts der Haut eines Individuums an vorgegebenen Inhaltsstoffen ergibt, daß der Hautstatus mehr oder weniger den idealen Verhältnissen einer gesunden Haut entspricht. In diesem Fall ergibt sich im zweiten Verfahrensschritt, daß die Abweichung der ermittelten Werte von den Standardwerten des Gehalts der Haut an vorgegebenen Inhaltsstoffen gering oder sogar gleich Null ist. In diesem Fall kann das Verfahren gemäß der Erfindung, insbesondere das kosmetische Verfahren, zu Pflege- oder Präventionszwecken einen oder mehrere Schritt(e) einer Supplementierung der Haut des Individuums mit Zubereitungen umfassen, die ein zukünftiges Abweichen der Werte des Gehalts der Haut des Individuums an den in Rede stehenden Inhaltsstoffen verhindern oder zumindest dazu beitragen, ein zukünftigen Abweichen zu verhindern.

Bei den vorgegebenen Inhaltsstoffen, die mit dem erfindungsgemäßen System ermittelt und bei Fehlen einzeln oder in einer Gruppe mehrerer Inhaltsstoffe supplementiert werden sollen, handelt es sich im weitesten Sinn um Inhaltsstoffe der Haut, insbesondere der menschlichen Haut. Insbesondere sind solche Inhaltsstoffe gemeint, die für die Wahrnehmung der natürlichen Funktionen der menschlichen Haut essentiell oder zumindest wünschenswert sind. Weiter bevorzugt sind solche Inhaltsstoffe, die der Fachmann als mit dem System der Erfindung ermittelbar bzw. supplementierbar kennt. Beispiele sind Proteine, Lipide, den Feuchtigkeitsgehalt der Haut steuernde Mittel, der Hautalterung vorbeugende Mittel, die Heilung der Haut nach Verletzungen etc. steuernde Mittel, die Durchblutung der Haut fördernde Mittel, den Schutz der Haut vor mechanischer oder Strahlenbelastung fördernde oder begünstigende Mittel, die Pigmentierung der Haut steuernde Mittel, Vitamine, Spurenelemente, Enzyme und Aminosäuren, ohne daß diese Aufzählung beschränkend verstanden werden soll. Besonders bevorzugt sind Lipide, an denen die Erfindung nachfolgend besonders erläutert wird, ohne darauf beschränkt zu sein.

Im Rahmen der Erfindung wurde überraschenderweise gefunden, daß im Gegensatz zu den Kenntnissen des Standes der Technik der Haut mit dem erfindungsgemäßen System eine einzelne fehlende Substanz oder nacheinander mehrere einzelne fehlende Substanzen oder mehrere Substanzen gemeinsam zugeführt werden können. In diesem Zusammenhang bedeute 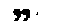 fehlend", daß die entsprechende(n) Substanz(en) der Haut völlig fehlt/fehlen oder diese Substanz(en) in der Haut nur in unzureichender Menge zugegen ist/sind. Mit dem erfindungsgemäßen System ist es möglich, diese Substanz(en) in beliebigen Mengen der Haut zuzuführen.

Der Weg der Zuführung ist im wesentlichen vom jeweiligen Fall abhängig und ist insbesondere danach auszuwählen, wie der Status der jeweiligen Haut bzw. Hautpartie des Individuums ist und welchen Zufuhrweg die jeweilige Substanz bzw. die Gruppe von Substanzen erlaubt bzw. wünschenswert macht. Hierbei sind dem Fachmann keine Beschränkungen auferlegt, solange der jeweilige Zufuhrweg die gezielte qualitative und quantitative Zufuhr der gewünschten Substanz(en) erlaubt.

### A) Mittel zur Ermittlung des Gehalts der Haut eines Individuums an vorgegebenen Inhaltsstoffen:

Voraussetzung für die Supplementierung der Haut eines Individuums mit Inhaltsstoffen ist die genaue Kenntnis des Hautstatus, d. h. die Kenntnis darüber, welche(r) Inhaltsstoff(e) der Haut zu einem bestimmten Zeitpunkt zur Verfügung steht/stehen, um ihre Funktionen ausführen zu können. Dafür stehen dem Fachmann eine ganze Anzahl verschiedener Möglichkeiten zur Verfügung.

Diese umfassen beispielsweise Systeme, mit denen die Haut mit einem mit einem Haftmittel versehenen Material in Kontakt gebracht wird, an dem Hautzellen der Hautoberfläche (Epidermiszellen) zum Haften gebracht werden können. Ein derartiges System umfaßt einen Streifen beispielsweise eines polymeren oder sonstigen geeigneten Trägermaterials, dessen eine Seite mit dem Haftmittel versehen ist. Die Haut wird mit diesem System für eine bestimmte Zeit in Kontakt gebracht, so daß eine ausreichende Menge von Hautzellen, die die zuverlässige Ermittlung des Gehalts der Haut des Individuums an vorgegebenen Inhaltsstoffen erlaubt, an dem Haftmittel haften bleibt und von der Haut entfernt wird. Die entfernten Zellen bzw. Partikel können dann hinsichtlich ihrer Zusammensetzung, insbesondere hinsichtlich ihres Gehalts an den vorgegebenen Inhaltsstoffen, analysiert werden.

Gemäß einer anderen beispielhaften Ausführungsform der Erfindung umfassen die Mittel zur Ermittlung des Gehalts der menschlichen Haut an vorgegebenen Inhaltsstoffen Systeme, mit denen aus der menschlichen Haut ein repräsentatives Spektrum von darin enthaltenen Inhaltsstoffen auf chemischem bzw. physikalischem Weg herausgelöst werden kann. Dies kann beispielsweise mit einem mit einem geeigneten Lösungsmitteln beaufschlagten Träger aus beispielsweise textilem Material oder einem anderen Material, einem beispielsweise relativ harten Material aus einem Polymer o. ä. geschehen. Aus den gefundenen Werten für die jeweiligen Substanzen und den bekannten statistischen Mittelwerten bzw. den gewünschten Werten der jeweiligen Substanzen kann dann ermittelt werden, welchen Bedarf die jeweilige Haut (-partie) an den fehlenden Substanzen hat.

### B) Mittel zum Vergleich der ermittelten Werte mit Standardwerten des Gehalts der Haut an vorgegebenen Inhaltsstoffen

Wie bereits oben erwähnt, sind gewisse Werte des Gehalts der menschlichen Haut an bestimmten Inhaltsstoffen bekannt, wünschenswert oder gar essentiell für ein zufriedenstellendes Funktionieren der Haut. Diese Werte sind beispielsweise tabellarisch oder in Form von elektronischen Dateien erfaßt und werden nun mit den Werten verglichen, die - wie vorstehend beschrieben - mit den oben genannten Mitteln ermittelt wurden. Beispielsweise können diese Mittel in elektronischen Dateien bestehen, in die entsprechenden Daten enthalten bzw. in die zum Vergleich die ermittelten Daten eingegeben werden. Andere, dem Fachmann bekannte Mittel können in gleicher Weise verwendet werden, solange sie für das Ziel brauchbar sind, einen Vergleich der ermittelten Werte mit Standardwerten bzw. gewünschten Werten des Gehalts der menschlichen Haut an vorgegebenen Inhaltsstoffen zu ermöglichen.

### C) Mittel zur Ableitung des Bedarfs der Haut des Individuums an den vorgegebenen Inhaltsstoffen aufgrund des Vergleichs der ermittelten Werte mit den Standardwerten

Im nächsten Schritt kommen Mittel zum Einsatz, mit denen der Bedarf der menschlichen Haut eines bestimmten Individuums an vorgegebenen Inhaltsstoffen ermittelt werden kann. Dies erfolgt bevorzugt mit Mitteln, die die Standardwerte bzw. eingestellten gewünschten Werte mit den wie oben beschrieben ermittelten Werten vergleichen. Ein solcher Vergleich kann beispielsweise durchgeführt werden aufgrund gewisser Erfahrungen in der Supplementierung der Haut des hier betrachteten Individuums mit den gewünschten oder essentiellen Inhaltsstoffen. Er kann jedoch in gleicher Weise durchgeführt werden aufgrund standardisierter Angaben, die in elektronischer oder tabellarischer Form gespeichert sind. Mit derartigen elektronischen oder tabellarischen Angaben ist ein schneller und bequemer Vergleich zur Ableitung des Bedarfs der menschlichen Haut des Individuums an den vorgegebenen Inhaltsstoffen möglich, der auch vom Laien ohne weiteres durchgeführt werden kann.

Beispielsweise können die in der Stufe der Ermittlung der Statuswerte des Gehalts der menschlichen Haut an bestimmten Inhaltsstoffen ermittelten Werte an den vorgegebenen Komponenten der Haut in vorgegebene Tabellen eingetragen und dann Standardwerte bzw. gewünschte Werte ausgerechnet werden. In gleicher Weise ist es möglich und entspricht einer weiteren Ausführungsform der Erfindung, die ermittelten Werte elektronisch zu verarbeiten und durch Vergleich der Ist-Werte mit den Soll-Werten bzw. gewünschten Werten zu ermitteln, welchen Bedarf die Haut an den jeweiligen Komponenten hat, um die Soll-Werte zu erreichen oder bestimmte Funktionen wieder oder wieder voll erfüllen zu können.

### D) Mittel zur Komposition einer Zubereitung, deren Zusammensetzung exakt auf den Bedarf der Haut an den vorgegebenen Inhaltsstoffen abgestimmt ist

Umfaßt von der Erfindung sind weiter Mittel zur Komposition einer Zubereitung, mit der der Haut bestimmte vorgegebene Inhaltsstoffe zugeführt werden können. Dies können einzelne Inhaltsstoffe aus einer vorgegebenen Gruppe oder mehrere derartige Inhaltsstoffe sein. Besonders bevorzugt sind es Inhaltsstoffe, die in Qualität und Quantität früher als Bedarf der individuellen menschlichen Haut an Substanzen zur Wiederherstellung der Hautfunktionen oder zum Erreichen bestimmter Wirkungen auf oder in der Haut ermittelt worden waren.

Die einzelnen Komponenten einer Zubereitung können dabei in Vorratsmengen bereitgestellt sein, um sie in dem ermittelten Verhältnis zueinander entnehmen und beispielsweise in einem Mischgefäß mischen zu können. Dabei können übliche Träger und Hilfsstoffe Verwendung finden, die in gleicher Weise bereitgestellt werden wie die genannten Komponenten. Die Entnahme und das Mischen können dabei in einer Ausführungsform der Erfindung manuell erfolgen, was erfahrungsgemäß zu hinreichenden Ergebnissen führt und die einfachste und am breitesten anwendbare Form der Komposition einer Zubereitung ist, mit der die Haut mit fehlenden Komponenten supplementiert werden kann.

In einer weiteren Ausführungsform der Erfindung ist es möglich, die Komposition einer bestimmte Komponenten enthaltenden Zubereitung einschließlich der Zufügung bestimmter Träger und/oder Hilfsstoffe mit Hilfe automatisierter Vorrichtungen durchzuführen, die mit den vorangehend beschriebenen Einrichtungen gekoppelt sind. Damit ist eine sofortige Verwertung der aus den oben beschriebenen Dateien stammenden Daten für die Herstellung der Zubereitungen möglich.

Derartige Zubereitungen können in einer Vielzahl von Formen wie beispielsweise Salben, Cremes, Lotionen, Wasser-in-Öl-Systemen oder Öl-in-Wasser-Systemen und Pudern vorliegen, ohne auf diese beschränkt zu sein. Derartige Zubereitungen können neben Inhaltsstoffen, die die Haut supplementieren sollen, auch noch andere Inhaltsstoffe enthalten. Als solche kommen übliche, dem Fachmann für solche Zwecke bekannte Träger und Hilfsstoffe, übliche, dem Fachmann für solche Zwecke bekannte Mittel zur Haltbarmachung und Konservierungsmittel, übliche, dem Fachmann für solche Zwecke bekannte kosmetische Hilfsstoffe und Wirkstoffe, Duftstoffe, Farbstoffe usw. infrage. Sie können rein kosmetischen Zwecken dienen und damit auch alle Eigenschaften haben, die entsprechende herkömmliche Kosmetika aufweisen, oder auch ergänzend oder ausschließlich medizinischen Zwecken dienen und damit auch die Haut heilende Funktionen haben. Im Vordergrund steht jedoch, die Haut mit Wirkstoffen zu supplementieren, die die Haut wieder in den Zustand setzen, ihre natürlichen Funktionen wieder wahrnehmen zu können.

### E) Mittel zur Supplementierung der Haut des Individuums mit der komponierten Zubereitung

Weiter umfaßt die Erfindung Mittel, mit denen die Haut des Individuums mit der komponierten Zubereitung supplementiert werden kann, um die Inhaltsstoffe mit der Haut in Kontakt zu bringen und diese zu veranlassen, in die Haut einzudringen, wo sie ihre Wirkung entfalten. Derartige Mittel können in geeigneten Gefäßen bestehen, in denen die Zubereitungen entweder mit allen Komponenten gemeinsam enthalten sind oder in Gefäßen, in denen einzelne Komponenten oder Gruppen von Komponenten enthalten sind, die - um Probleme der Langzeitlagerung zu vermeiden - erst kurz vor der Supplementierung vom Anwender in Mengen miteinander vermischt werden, die für eine Applikation ausreichend sind. Derartige Gefäße können mit geeigneten Applikatoren versehen sein, die eine Applikation auf bestimmte Partien der Haut ermöglichen oder auch nur erleichtern. Derartige Applikatoren haben darüber hinaus den Vorteil, daß sie eine exakte Portionierung der Zubereitung erlauben und damit ermöglichen, daß der Haut des Individuums exakt die richtige Menge der Zubereitung zugeführt wird.

Mit den erfindungsgemäßen Mitteln ist es also möglich, auf bestimmte Erfordernisse der Haut zu reagieren und einzelne Komponenten oder eine Mehrzahl von Komponenten in einer Weise der Haut zuzuführen, die eine exakte Supplementierung mit den erforderlichen bzw. gewünschten Komponenten erlaubt. Überraschenderweise ergibt sich dabei, daß nicht nur kosmetischen Erfordernissen genügt werden kann, sondern die Haut insbesondere mit solchen Komponenten einzeln oder in einer Zusammenstellung mehrerer Komponenten versorgt werden kann, die eine Einstellung oder Wiederherstellung der natürlichen Hautfunktionen bereits nach wenigen Behandlungen ermöglicht. Weitere Vorteile sind darin zu sehen, daß mit dem erfindungsgemäßen Mittel bzw. Verfahren, insbesondere mit dem kosmetischen Verfahren, auch einem präventiven Hautschutz gegen externe Belastungen der Haut Rechnung getragen wird. Weiter kann bei Anwendung der erfindungsgemäßen Mittel bzw. bei Durchführung des erfindungsgemäßen Verfahrens nach Prozessen der Heilung der Haut von einer ganzen Reihe chronischer Hauterkrankungen die gestörte Barriere-Funktion der Haut regeneriert werden.

Die Erfindung wird nachfolgend unter Bezugnahme auf das folgende Ausführungsbeispiel erläutert, ohne auf dieses beschränkt zu sein.

### Ausführungsbeispiel

Im ersten Schrift wurde an einem Probanden Probenmaterial gewonnen. Dies geschah lokal auf einem Hautareal einer Größe von 5 cm² entweder mittels Auswaschen (mit einem Gemisch aus Aceton und Ether) oder mittels Hornschichtabrissen (mit einem Klebestreifen).

An dem so erhaltenen Probematerial wurde nach an sich bekannter Vorbereitung eine Lipidanalyse mittels HPLC durchgeführt. Hauptparameter waren Ceramide, Cholesterin und freie Fettsäuren. Aus den Ergebnissen wurde ein individuelles Lipid-Diagramm erstellt, das die individuelle Lipid-Zusammensetzung für dieses Hautareal zum Zeitpunkt der Probenentnahme widerspiegelte.

Im Vergleich mit einem Standard-Lipid-Diagramm wurden Abweichungen des individuellen Lipid-Diagramms ermittelt.

Unter Berücksichtigung der so ermittelten Abweichungen wurde eine Creme hergestellt, die die fehlenden Lipidkomponenten in den Mengen enthielt, die sich aus der Abweichung des individuellen Lipidmusters von dem Standard-Lipidmuster ergaben. Dabei wurden kommerziell verfügbare Lipide in der jeweiligen durch Differenzbildung ermittelten Menge in eine für Externa geeignete Galenik eingearbeitet.

Die so erhaltene Crême wurde auf ein bezüglich der Fläche exakt definiertes Hautareal aufgetragen, um eine mengenmäßig genaue Supplementierung der Haut mit den fehlenden Lipiden zu erreichen.

Bei Wiederholung des Vorgehens nach einer wie vorstehend beschrieben durchgeführten Verfahrensweise ergab sich, daß die Abweichungen der individuellen Lipidwerte von den Werten des Standard-Lipidmusters geringer waren und in einem Fall sogar nur noch im Bereich einer statistischen Streuung lagen.

In einem weiteren Fall wurden individuelle Lipidwerte mit dem vorstehend beschriebenen Verfahren ermittelt, die im Bereich der Werte des Standard-Lipidmusters lagen (Abweichungen - sofern vorhanden - im Bereich der statistischen Streuung). In diesem Fall erfolgte eine Komposition einer Crême mit einer Zusammensetzung, die sich im wesentlichen an dem Standard-Lipidmuster orientierte.

Bei Behandlung mit einer derartigen Crême ergaben sich über die Zeit keine Abweichungen des individuellen Lipidmusters von dem Standard-Lipidmuster selbst bei Belastung der beobachteten Hautpartien.

Wie aus dem vorliegenden Ausführungsbeispiel zu entnehmen ist, ist wesentliches Element der Erfindung das Mittel zur Ermittlung des Gehalts der Haut (hier: der menschlichen Haut) an vorgegebenen Inhaltsstoffen (hier: an Lipiden der Haut). Erst nach der Ermittlung kann im Vergleich zu bekannten Standardwerten der jeweiligen Gehalte der Haut abgeleitet werden, mit welchen Inhaltsstoffen die Haut supplementiert werden muß. Im Unterschied zum Stand der Technik, in dem die Haut mit standardisiert zusammengesetzten Zubereitungen behandelt wurde, die nicht auf den aktuellen Hautstatus abgestellt waren, kann mit dem erfindungsgemäßen System und dem erfindungsgemäßen Verfahren, insbesondere mit dem kosmetischen Verfahren, dem aktuellen individuellen Status der Haut des einzelnen Individuums gezielt Rechnung getragen werden, was den Behandlungserfolg wesentlich verbessert.

## Patentansprüche

1. System zur Ermittlung des Gehalts der Haut, insbesondere der menschlichen Haut, an verschiedenen, insbesondere an für die Funktionen der Haut essentiellen, Inhaltsstoffen und zur an das Ergebnis der Ermittlung angepaßten Supplementierung mit den fehlenden Inhaltsstoffen, gekennzeichnet durch
- Mittel zur Ermittlung des Gehalts der Haut eines Individuums an vorgegebenen Inhaltsstoffen;
- Mittel zum Vergleich der ermittelten Werte mit Standardwerten des Gehalts der Haut an vorgegebenen Inhaltsstoffen;
- Mittel zur Ableitung des Bedarfs der Haut des Individuums an den vorgegebenen Inhaltsstoffen aufgrund des Vergleichs der ermittelten Werte mit den Standardwerten;
- Mittel zur Komposition einer Zubereitung, deren Zusammensetzung exakt auf den Bedarf der Haut an den vorgegebenen Inhaltsstoffen abgestimmt ist; und
- Mittel zur Supplementierung der Haut des Individuums mit der komponierten Zubereitung.

2. System nach Anspruch 1 für medizinische Zwecke.

3. System nach Anspruch 1 für kosmetische Zwecke.

4. System nach einem der Ansprüche 1 bis 3 in Form von Salben, Crêmes, Lotionen, Wasser-in-Öl-Systemen oder Öl-in-Wasser-Systemen und Pudern.

5. Verfahren zur Behandlung und/oder Pflege der Haut und/oder zur Vorbeugung von Mangelerscheinungen daran, bei dem man
- den Gehalt der Haut eines Individuums an vorgegebenen Inhaltsstoffen ermittelt;
- die ermittelten Werte mit Standardwerten des Gehalts der Haut an vorgegebenen Inhaltsstoffen vergleicht;
- aufgrund des Vergleichs der ermittelten Werte mit den Standardwerten den Bedarf des Individuums an den vorgegebenen Inhaltsstoffen ableitet;
- eine Zubereitung zusammenstellt, deren Zusammensetzung exakt auf den Bedarf der Haut an den vorgegebenen Inhaltsstoffen abgestimmt ist; und
- die Haut des Individuums mit der zusammengestellten Zubereitung supplementiert.

6. Verfahren nach Anspruch 5 für medizinische Zwecke, insbesondere zur Behandlung von Hautkrankheiten.

7. Verfahren nach Anspruch 5 für kosmetische Zwecke, insbesondere zur Pflege der Haut.
